(19) Europäisches Patentamt
European Patent Office
Office européen des brevets

(11) Publication number: **0 524 671 A1**

(12) # EUROPEAN PATENT APPLICATION

(21) Application number: **92201809.8**

(22) Date of filing: **19.06.92**

(51) Int. Cl.5: **C12N 1/14**, C12P 7/40, //(C12P7/40,C12R1:645)

(30) Priority: **27.06.91 US 722049**

(43) Date of publication of application: **27.01.93 Bulletin 93/04**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Diez, Maria Teresa**
**c/Olid, 4**
**E-28010 Madrid(ES)**
Inventor: **Bills, Gerald F.**
**402 Alden Road**
**Roselle, NJ 07203(ES)**
Inventor: **Kong, Yu Lin**
**57 Bryant Avenue**
**Edison, NJ 08820(ES)**
Inventor: **Omstead, Mary Nallin**
**13 Deer Path**
**Gladstone, NJ 07934(ES)**
Inventor: **Pelaez, Fernando**
**C/Camino de Valderribas 10**
**E-28038 Madrid(ES)**

(74) Representative: **Thompson, John Dr. et al**
**Merck & Co., Inc. European Patent**
**Department Terlings Park Eastwick Road**
**Harlow, Essex CM20 2OR(GB)**

(54) **Novel culture of exserohilum rostratum and processes therefrom.**

(57) This invention relates to a strain of Exserohilum rostratum isolated from tree bark and useful in a fermentation process to form compounds of formula (I):

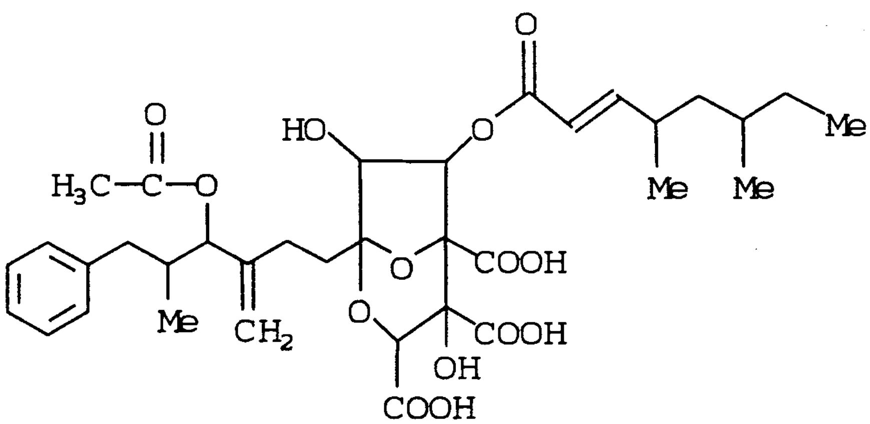

(I)



Rank Xerox (UK) Business Services

FIGURE 1

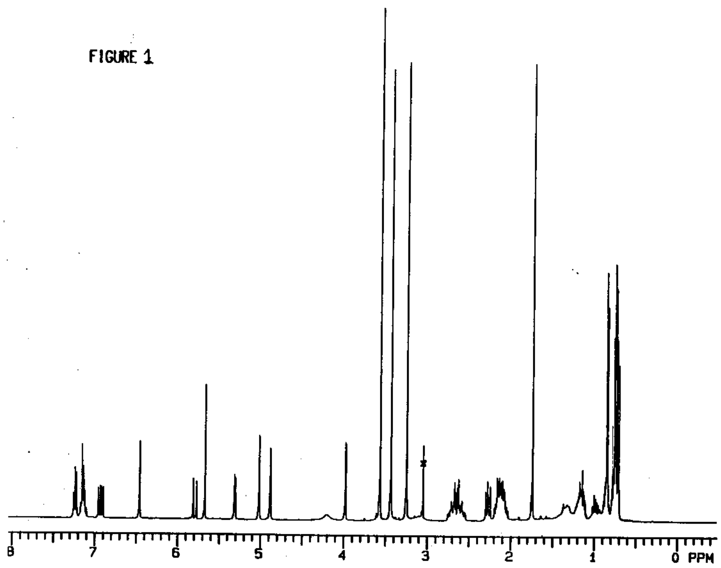

BACKGROUND OF THE INVENTION

Hypercholesterolemia is known to be one of the prime risk factors for ischemic cardiovascular disease, such as arteriosclerosis. Bile acid sequestrants have been used to treat this condition; they seem to be moderately effective but they must be consumed in large quantities, i.e. several grams at a time and they are not very palatable.

MEVACOR® (lovastatin), now commercially available, is one of a group of very active antihypercholesterolemic agents that function by limiting cholesterol biosynthesis by inhibiting the enzyme, HMG-CoA reductase.

Squalene synthetase is the enzyme involved in the first committed step of the de novo cholesterol biosynthetic pathway. This enzyme catalyzes the reductive dimerization of two molecules of farnesyl pyrophosphate to form squalene. The inhibition of this committed step to cholesterol should leave unhindered biosynthetic pathways to ubiquinone, dolichol and isopentenyl t-RNA.

Previous efforts at inhibiting squalene synthetase have employed pyrophosphate or pyrophosphate analog containing compounds such as those described in P. Ortiz de Montellano et al, J. Med. Chem. 20, 243 (1977) and E.J. Corey and R. Volante, J. Am. Chem. Soc., 98, 1291 (1976). S. Biller (U.S. Patent 4,871,721) describeds isoprenoid (phosphinylmethyl)phosphonates as inhibitors of squalene synthetase.

SUMMARY OF THE INVENTION

The present invention provides a strain of Exserohilum rostratum that produces nonphosphorous containing inhibitors of squalene synthetase.

DETAILED DESCRIPTION OF THE INVENTION

The present invention is directed to a novel microorganism that produces compounds of structural formula (I) that are squalene synthetase inhibitors;

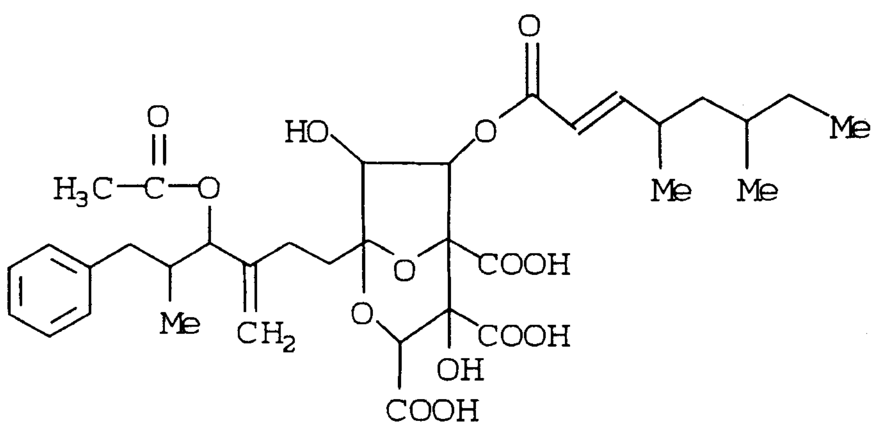

(I)

Compounds of formula (I) are useful as cholesterol-lowering agents and antifungal agents.

The compounds of formula (I) are prepared in an aerobic fermentation of Exserohilum rostratum. More particularly the strain employed may be strain MF5565, or a mutant organism thereof.

The culture MF5565, was isolated from bark of Theobroma cacao (Philippines). The culture has been deposited with the American Type Culture Collection (ATCC) at 12301 Parklawn Drive, Rockville, MD 20852 as ATCC 74068.

A biologically pure culture of Exserohilum rostratum as claimed herein is defined as being isolated from the natural environment and free of viable contaminating microorganisms. A culture of Exserohilum rostratum as claimed herein is defined as being isolated from the natural environment and free of viable contaminating culture that would be deleterious to the formation of a compound of formula (I). An active mutant of a microorganism as described herein is defined as a culture having one or more mutations and capable of producing compounds of formula (I).

This strain, MF5565, was recovered from the bark of Theobroma cacao, collected in Los Banos, Laguna Province, Philippines. Bark discs were removed with a leather punch (no. 245, C.S. Osborne & Co., Harrison, NJ). Discs were approximately 1 cm in diameter and 0.3-1.0 cm thick depending on the thickness of the bark and amount of force used to hammer the punch into the tree. Discs included an entire bark cross-section along with the vascular cambium, and sometimes a veneer of the outer xylem. Discs from each tree were placed in manila coin envelopes for transport to the laboratory. Discs were soaked in 10% household bleach for 3 minutes, rinsed with sterile distilled water and briefly flamed with an alcohol lamp prior to application to isolation media. Bark discs were applied outer side down to an agar media (10 g malt extract, 2 g yeast extract, 1 g sodium propionate, 5 g dehydrated bovine bile, 1 mg benomyl, 50 mg streptomycin sulfate, 50 mg chlorotetracycline, 20 g agar in 1 L distilled water) in 100 mm diameter plastic Petri dishes. Petri dishes were incubated at 24° C, and inspected more or less daily for up to one month for the development of fungal colonies on bark discs and the agar.

Strain MF5565 exhibits the following morphological characteristics.

Colonies relatively fast-growing, in 1 week attaining a diameter of: 50 mm on cornmeal agar (Difco Laboratories); 50-52 mm on yeast-malt extract agar (10 g malt extract, 2 g yeast extract, 20 g agar in 1 L distilled water); 60 mm on V8 juice agar (200 mL V8 juice, Campbell Soup Co., 3 g $CaCO_3$, 20 g agar diluted to 1 L distilled water). On yeast-malt agar with both submerged and aerial mucelium, with submerged mycelium sometimes forming radial strands, floccose to cottony or lanose in age, with margin appressed, minutely fimbriate to even, hyaline to pale gray at the margin but soom darkening to dark gray or dark olive-gray, or black in age, Dark Olive-Gray, Iron Gray, Dark Mouse Gray, Dusky Green-Gray, Blackish Green-Gray, Olivaceous Black (capitalized color names from Ridgway, R. 1912. Color Standards and Nomenclature, Washington, D.C.), similar in reverse, often with patches or tufts of hyaline to pale gray aerial hyphae developing in older portions. Odors, sclerotia, stromata, or pseudothecia absent. Conidiophores arising from uppermost aerial mycelium, up to 600 µm long, 3-4.5 µm wide, straight or flexuous, with geniculate apices, with walls smooth, or occasionally finely incrusted, usually bearing 2-10 conidia, pale olive-gray to olive-gray. Conidiogenous cells polytretic, integrated, sympodial, indeterminate, terminal or intercalary, with slightly raised, darkened scars surrounding a minute pore at the conidiogenous locus. Conidia 45-250 x 7-20 µm, mostly 75-180 µm long, variable in shape, broadly elliposidal, fusoid, obclavate, or tapered cylindrical, straight to curved, or rarely sigmoid, with broadly rounded apices, smooth, 5-22 septate, with basal septum most thickened and darkened, with terminal septum often also darker than septa delimiting central cells, with a distinct cylindrical hilar appendix protruding 1-2.5 µm, pedicel-like extensions absent, initially germinating from apical and basal cells. pale gray to olive-gray in 3% KOH. Hyphae septate, branched, pale olive-gray to olive-brown, usually smooth, but occasionally with fine incrustations.

Strain MF5565 belongs to the genus Exserohilum rostratum based on the combination of polytretic conidiogenous cells that give rise to predominately multiseptate, dematiaceous phragmoconidia. The basal cell of the conidium is delimited by a thick, darkened septum, and has a protruding hilar appendix. Strain MF5565 is identified as Exserohilum rostratum based on the predominance of straight and curved conidia, darkened septa delimiting both the basal and terminal cells, and relatively long conidia (A. Sivanesan. 1987 Graminicolous species of Bipolaris, Curvularia, Drechslera, Exserohilum and their telemorphs. CMI Mycological Paper No. 158).

Compounds of formula (I) can be obtained by cultivation of this microorganism in an aqueous nutrient medium containing sources of assimilable carbon and nitrogen, preferably under aerobic conditions. Nutrient media may also contain mineral salts and defoaming agents.

The preferred sources of carbon in the nutrient medium are carbohydrates such as glucose, glycerin, starch, dextrin, and the like. Other sources which may be included are maltose, mannose, sucrose, and the like. In addition, complex nutrient sources such as oat flour, corn meal, millet, corn and the like may supply utilizable carbon. The exact quantity of the carbon source which is used in the medium will depend, in part, upon the other ingredients in the medium, but is usually found in an amount ranging between 0.5 and 5 percent by weight. These carbon sources can be used individually in a given medium or several sources in combination in the same medium.

The preferred sources of nitrogen are amino acids such as glycine, methionine, proline, threonine and the like, as well as complex sources such as yeast extracts (hydrolysates, autolysates), dried yeast, tomato paste, soybean meal, peptone, corn steep liquor, distillers solubles, malt extracts and the like. Inorganic nitrogen sources such as ammonium salts (e.g., ammonium nitrate, ammonium sulfate, ammonium phosphate, etc.) can also be used. The various sources of nitrogen can be used alone or in combination in amounts ranging between 0.2 to 90 percent by weight of the medium.

The carbon and nitrogen sources are generally employed in combination, but need not be in pure form. Less pure materials which contain traces of growth factors, vitamins, and mineral nutrients may also be

used. Mineral salts may also be added to the medium such as (but not limited to) calcium carbonate, sodium or potassium phosphate, sodium or potassium chloride, magnesium salts, copper salts, cobalt salts and the like. Also included are trace metals such as manganese, iron, molybdenum, zinc, and the like. In addition, if necessary, a defoaming agent such as polyethylene glycol or silicone may be added, especially if the culture medium foams seriously.

The preferred process for production of compounds of this invention consists of inoculating spores or mycelia of the producing organism into a suitable medium and then cultivating under aerobic condition.

The fermentation procedure generally is to first inoculate a preserved source of culture into a nutrient seed medium and to obtain, sometimes through a two step process, growth of the organisms which serve as seeds in the production of the active compounds. After inoculation, the flasks are incubated with agitation at temperature ranging from 20 to 30°C, preferably 25 to 28°C. Agitation rates may range up to 400 rpm, preferably 200 to 220 rpm. Seed flasks are incubated over a period of 2 to 10 days, preferably 2 to 4 days. When growth is plentiful, usually 2 to 4 days, the culture may be used to inoculate production medium flasks. A second stage seed growth may be employed, particularly when going into larger vessels. When this is done, a portion of the culture growth is used to inoculate a second seed flask incubated under similar conditions but employing shorter time.

After inoculation, the fermentation production medium is incubated for 3 to 50 days, preferably 14 to 35 days, with or without agitation (depending on whether liquid or solid fermentation media are employed). The fermentation is conducted aerobically at temperatures ranging from 20 to 40°C. If used, agitation may be at a rate of 200 to 400 rpm. To obtain optimum results, the temperatures are in the range of 22 to 28°C, most preferably 24 to 26°C. The pH of the nutrient medium suitable for producing the active compounds is in the range of 3.5 to 8.5, most preferably 4.5 to 7.0. After the appropriate period for production of the desired compound, fermentation flasks are harvested and the active compound isolated.

An alcoholic solvent, possibly mixed with an oxygenated solvent, such as an ester or a ketone, is employed to extract compound (I) from solid fermentation medium.

The pH of the aqueous mycelial fermentation is adjusted to between 1 and 9 (preferably between 3 and 5) preferably mixed with a water miscible solvent such as methanol and the mycelia filtered. The active compound may then be isolated from the aqueous filtrate by several methods including:

1. Liquid-liquid extraction of the aqueous filtrate into a water immiscible solvent such as methyl ethyl ketone, ethyl acetate, diethyl ether, or dichloromethane preferably after having adjusted the pH to between 1.5 and 2.5.

2. Solid-liquid extraction of the aqueous filtrate onto an organic matrix such as SP207 or HP-20 and elution with an organic solvent (aqueous or non aqueous) such as 90/10 methanol/water or 90/10 acetone/water.

3. Adsorption of the active compound from the aqueous filtrate onto an ionic exchange resin such as Dowex 1($Cl^-$) and elution with a high ionic strength organic/aqueous solvent such as 90/10 methanol/aqueous 30% $NH_4Cl$. This material could then be desalted by employing either method 1 or 2 above. Each of these three methods may also be used in the further purification of the active compound. The active compound may also be adsorbed onto an ionic exchange resin such as BioRad AG4X4 from an aqueous filtrate and eluted with a solution of 0.2 N $H_2SO_4$ in 6/4 $CH_3CN/H_2O$. This material may then be desalting by either method 1 or method 2 above.

The fraction containing active compound from the above methods could then be dried in vacuo leaving the crude active compound. The crude active compound is then generally subjected to several separation steps such as adsorption and partition chromatography, and precipitation. For each separation step, fractions are collected and combined based on results from an assay and/or HPLC analysis.

The chromatographic separations may be carried out by employing conventional column chromatography with ionic or nonionic adsorbent. When silica gel is the adsorbent, an alcohol/chlorohydrocarbon/organic acid mixture such as methanol/chloroform/acetic acid/ water is useful as an eluant. For reverse phase chromatography, the preferred adsorbent is a $C_8$ or $C_{18}$ bonded phase silica gel. The preferred eluant for reverse phase chromatography is a mixture of acetonitrile and water buffered at a low pH, such as 0.1% phosphoric acid, or trifluoroacetic acid. The active compound can be precipitated out of a non-polar solvent as the quinine salt. The preferred solvent for precipitation is diethyl ether. The active compound (I) can also be precipitated out of polar solvents, such as methanol, as the ammonium salt.

The intrinsic squalene synthetase inhibitory activity of the compounds formed in the fermentation of the microorganisms of the present invention may be measured by the standard in vitro protocol described below:

Preparation of Rat Liver Microsomes:

Male, Charles River CD rats (120 to 150 g) were fed a diet containing 0.1% lovastatin for 4 days. The livers from these rats were homogenized in 5 volumes (mL/g) of ice cold 50 mM HEPES (4-(2-hydroxyethyl)-1-piperazine-ethanesulfonic acid), 5 mM EDTA(ethylenediaminetetraacetic acid) pH 7.5 with a Potter-Elvehjem type tissue grinder. The homogenate was centrifuged twice at 20,000 x g for 15 min. at 4° C, discarding the pellet each time. The supernatant was then centrifuged at 100,000 x g for 1 hr at 4° C. The resulting microsomal pellet was resuspended in a volume of the above homogenizing buffer equal to one-fifth the volume of the original homogenate. This microsomal preparation has a protein concentration of about 7 mg/mL. The microsomal suspensions were stored in aliquots at -70° C. Squalene synthetase activity in these aliquots is stable for at least several months.

Partial Purification of Prenyl Transferase

Prenyl transferase was purified to use in the enzymatic synthesis of radiolabelled farnesyl pyrophosphate. Prenyl transferase was assayed by the method of Rilling (Methods in Enzymology 110, 125-129 (1985)) and a unit of activity is defined as the amount of enzyme that will produce 1 μmole of farnesyl pyrophosphate per minute at 30° C in the standard assay.

The livers of 23 forty-day old male rats that had been fed 5% cholestyramine plus 0.1% lovastatin were homogenized in a Waring blender in 1 liter of 10 mM mercaptoethanol, 2 mM EDTA, 25 mM leupeptin, 0.005% phenylmethylsulfonyl fluoride, pH 7.0 containing 0.1 trypsin inhibitor units of aprotinin/mL. The homogenate was centrifuged at 20,000 x g for 20 min. The supernatant was adjusted to pH 5.5. with 6 N HOAc and centrifuged at 100,000 x g for 1 hour. This supernatant was adjusted to pH 7.0 with 3 N KOH and a 35-60% ammonium sulfate fraction taken. The 60% pellet was redissolved in 60 mL of 10 mM potassium phosphate, 10 mM mercaptoethanol, 1 mM EDTA pH 7.0 (Buffer A) and dialyzed against two 1 liter changes of Buffer A. This dialyzed fraction was applied to a 12.5 x 5 cm column of DEAE-sepharose 4B equilibrated with Buffer A. The column was washed with 700 mL of Buffer A and a 1 liter gradient from Buffer A to 100 mM potassium phosphate, 10 mM mercaptoethanol, 1 mM EDTA, pH 7.0. Fractions having a specific activity greater than 0.20 units/mg were combined, solid ammonium sulfate was added to bring to 60% saturation and pelleted. The pellet was dissolved in 8 ml of 10 mM Tris, 10 mM β-mercaptoethanol pH 7.0 (Buffer B). The redissolved pellet was taken to 60% saturation with ammonium sulfate by adding 1.5 volumes of saturated ammonium sulfate in Buffer B. This ammonium sulfate suspension contained 3.5 units/mL with a specific activity of 0.23 units/mg and was free of isopentenyl pyrophosphate isomerase activity. This ammonium sulfate suspension was used for the synthesis of [4-$^{14}$C]farnesyl-pyrophosphate and its activity was stable stored at 4° C for at least 6 months.

Enzymatic Synthesis of [4-$^{14}$C]farnesyl-pyrophosphate

The solvent (ethanol: 0.15 N $NH_4OH$, 1:1) was removed from 55 mCi of [4-$^{14}$C]isopentenyl pyrophosphate(47.9 mCi/mmole) by rotary evaporation. Six hundred microliters of 100 mM Tris, 10 mM $MgCl_2$, 4 mM dithiothreitol pH 7.5 was added and the solution was transferred to a 1.5 mL Eppendorf centrifuge tube. Geranyl-pyrophosphate, 250 mL of a 20 mM solution, and 50 mL of the ammonium sulfate suspension of prenyl transferase were added to initiate the reaction. This incubation contained 5 mmoles of geranyl pyrophosphate, 1.15 mmoles of isopentenyl pyrophosphate, 6 mmoles of $MgCl_2$ of 0.18 units of prenyl transferase in a volume of 900 mL. The incubation was conducted at 37° C. During the incubation, the mix turned cloudy white as the newly formed magnesium complex of farnesyl pyrophoshate precipitated out of solution. The [4-$^{14}$C]farnesyl pyrophosphate was collected by centrifugation for 3 minutes at 14,000 rpm in an Eppendorf centrifuge tube, the supernatant removed, and the pellet was dissolved in 1.0 mL of 50 mM HEPES, 5 mM EDTA, pH 7.5 The yield was 50.7 mCi (92%) of [4-$^{14}$C]farnesyl pyrophosphate. The [4-$^{14}$C]farnesyl pyrophosphate was stored in aliquots at -70° C.

Squalene Synthetase Assay

Reactions were performed in 16 x 125 mm screw cap test tubes. A batch assay mix was prepared from the following solution:

| | mL per assay | volume for 50 assays |
|---|---|---|
| 1. 250 mM HEPES pH 7.5 | 20 | 1000 |
| 2. NaF 110 mM | 10 | 500 |
| 3. $MgCl_2$ 55 mM | 10 | 500 |
| 4. Dithiothreitol 30 mM | 10 | 500 |
| 5. NADPH 10 mM (made fresh) | 10 | 500 |
| 6. [4-$^{14}$C]farnesyl-pyrophosphate 47.9 mCi/mmole, and 0.025 mCi/3.0 mL | 3.0 | 150 |
| 7. $H_2O$ | 24 | 1200 |

This assay mix was degassed under a vacuum and flushed with $N_2$. Solutions of the squalene synthetase inhibitors were prepared either in DMSO or MeOH and a 1:120 dilution of the microsomal protein was made with the original homogenizing buffer. For each reaction, 87 mL of the assay mix was taken with 3 mL of an inhibitor solution (DMSO or MeOH in the controls), warmed to 30°C in a water bath and then the reaction was initiated by the addition of 10 mL of the 1:120 dilution of microsomal protein (0.6 mg protein total in the assay). The reactions were stopped after 20 minutes by the addition of 100 mL of a 1:1 mix of 40% KOH with 95% EtOH. The stopped mix was heated at 65°C for 30 min., cooled, 10 mL of heptane was added and the mix was vortexed. Two g of activated alumina was then added, the mix vortexed again, the alumina allowed to settle and 5 mL of the heptane layer was removed. Ten mL of scintillation fluid was added to the heptane solution and radio-activity was determined by liquid scintillation counting.

Percent inhibition is calculated by the formula:

$$1 - \frac{[\text{Sample} - \text{Blank}]}{[\text{Control} - \text{Blank}]} \times 100$$

The composition of media employed in the following Examples are listed below:

| YME Plating Medium | |
|---|---|
| Component | Amount |
| Yeast Extract | 4.0 g |
| Malt Extract | 10.0 g |
| Glucose | 4.0 g |
| Distilled $H_2O$ | 1000 mL |
| Agar | 25.0 g |

**KF SEED MEDIUM**

| | per liter |
|---|---|
| Corn Steep Liquor | 5 g |
| Tomato Paste | 40 g |
| Oat Flour | 10 g |
| Glucose | 10 g |
| Trace Element Mix | 10 mL |

pH adjusted to 6.8 (presterile)
50 mL/nonbaffled 250 mL Erlenmeyer flask
autoclave 20 min. (121°C, 15 psi)

**Trace Element Mix**

| | g/L |
|---|---|
| $FeSO_4 \cdot 7H_2O$ | 1.0 |
| $MnSO_4 \cdot 4H_2O$ | 1.0 |
| $CuCl_2 \cdot 2H_2O$ | 0.025 |
| $CaCl_2 \cdot 2H_2O$ | 0.1 |
| $H_3BO_3$ | 0.056 |
| $(NH_4)_6Mo_7O_{24} \cdot 4H_2O$ | 0.019 |
| $ZnSO_4 \cdot 7H_2O$ | 0.2 |

dissolved in 1 L 0.6 N HCl

Production Medium

F204

Prepared in nonbaffled 250 mL Erlenmeyer flasks

Millet - 15.0 g/flask
Base Liquid #2 - 10.0 mL/flask

| Base Liquid #2 | |
|---|---|
| | g/L |
| Yeast extract | 50.0 |
| Monosodium glutamate | 10.0 |
| Corn oil | 10.0 mL |
| Sodium tartrate | 10.0 |
| $FeSO_4 \cdot 7H_2O$ | 1.0 |
| Distilled $H_2O$ | 1000 mL |
| no pH adjustment<br>autoclave 15 minutes (121 ° C, 15 psi)<br>add 15.0 mL distilled $H_2O$/flask<br>autoclave 20 minutes (121 ° C, 15 psi) | |

The following example illustrates the preparation of compounds of formula (I) and is not to be considered as limiting the invention set forth in the claims hereto.

Example 1

A. Culturing MF5565

Culture MF5565 was inoculated into KF seed medium using one glass scoop of a mixture of spores and

hyphae that had been preserved in sterile soil. The KF seed flask was incubated for 75 hours at 25°C, 220 rpm, and 85% humidity. At the end of this incubation, 2.0 mL aliquots were aseptically transfered to each of 16 F204 solid production medium flasks. These production flasks were then incubated at 25°C statically for 33 days. At harvest 45 mL of 70% methanol were added to each flask and the solid growth was manually broken apart into smaller pieces. Flasks were then placed onto a gyrotory shaker and agitated at 220 rpm for 30 minutes in order to further break up the mycelial mass as well as to improve contact of the solvent with the cells. After shaking, the contents of the individual flasks were pooled by pouring the entire contents of the flasks (solids and all) into a 1.5 L beaker.

B. Isolation of Compound I

A methanol extract of the fermentation of Example 1, Part A corresponding to 86 mL of whole broth was adjusted to 50 mM NaOAc by addition of 10 mL of a pH 4.7 solution of 1.0 M sodium acetate. The buffered extract was then adjusted to pH 4.6 with glacial acetic acid. BioRad AG4X4 resin (200-400 mesh, free base form, 10 g) was added and the solution was stirred for 4.5 hr at room temperature. The resin was then transferred to a column (volume = 22 mL, 2.5 cm x 4.0 cm) and washed with 50 mL of a solution of 6/4 $CH_3CN/H_2O$. The column was then eluted with a solution of 0.1 N $H_2SO_4$ in 6/4 $CH_3CN/H_2O$ collecting 7.5 mL fractions. Fractions 21 - 55 were pooled and contained compound I as determined by HPLC analysis. The pooled fractions (260 mL) were extracted with 100 mL of ethyl acetate and the ethyl acetate layer concentrated in vacuo.

The desalted AG4x4 eluate above was dissolved in 8.3 mL methanol. A 1 mL portion of this solution was concentrated to 0.5 mL and subjected to HPLC (Phenomenex Ultracarb 5 ODS 30, 9.4 mm x 25 cm, gradient elution 35% to 100% solvent B, solvent A = 40% $CH_3CN$/60% $H_2O$ + 0.1% $H_3PO_4$, solvent B = 75% $CH_3CN/H_2O$ + 0.1% $H_3PO_4$, flow rate 3.0 mL/min, Waters 990+ diode array detection) and 3 mL fractions were collected. Fraction 31 contained essentially pure Compound I as determined by analytical HPLC (Phenomenex Ultracarb 5 ODS 30, 15 cm x 4.6 mm, 0.1% $H_3PO_4$ in 65/35 $CH_3CN/H_2O$, flow rate 1.0 mL/min, UV detection at 210 nm) retention time and UV comparison with authentic standard. One half (1.5 mL) of fraction 31 was concentrated to 0.75 mL under a stream of nitrogen, 0.75 mL of $H_2O$ was added and then the mixture was extracted with 1.5 mL $CH_2Cl_2$. The $CH_2Cl_2$ layer was dried over anhydrous sodium sulfate and concentrated to dryness. The sample was then dissolved in 1 mL diethyl ether. The sample was treated with ethereal diazomethane at 0 degrees C for 5 minutes to form the trimethyl ester of Compound I. The reaction mixture was concentrated to dryness under a stream of nitrogen and subjected to FAB MS analysis. A molecular weight of 732 was found corresponding to the molecular ion of the trimethyl ester of Compound I.

C. Physical Properties of Compound I

Mass Spectral Data:

This compound has the molecular weight 732 by EI-MS and forms a di-(trimethylsilyl) derivative. The molecular formula $C_{38}H_{52}O_{14}$ was determined directly by HR-EI measurement (calc. 732.3357, found 732.3329).

$^1$H NMR Spectrum ($C_6D_6$, 22°C): See figure 1.

$^{13}$C NMR Chemical Shifts ($C_6D_6$, 75 MHz): 11.3, 14.0, 18.9, 20.2, 20.6, 26.3, 30.0, 32.1, 34.6, 34.7, 37.1, 40.2, 43.3, 51.9, 52.1, 53.3, 75.3, 76.2, 78.8, 82.0, 82.8, 89.8, 106.2, 111.8, 118.8, 126.2, 128.6(2x), 129.6-(2x), 140.9, 146.6, 157.2, 166.0, 166.5, 167.2, 169.5, 170.3 ppm.

**Claims**

**1.** A biologically pure culture of Exserohilum rostratum capable of producing a fermentation broth that inhibits squalene synthetase activity and which produces:

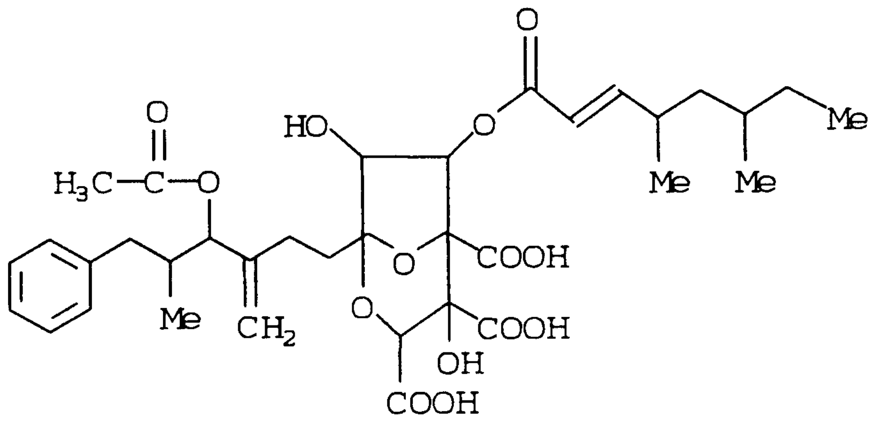

(I)

in recoverable amounts.

2. A culture of Exserohilum rostratum capable of producing a fermentation broth which inhibits squalene synthetase activity and which produces:

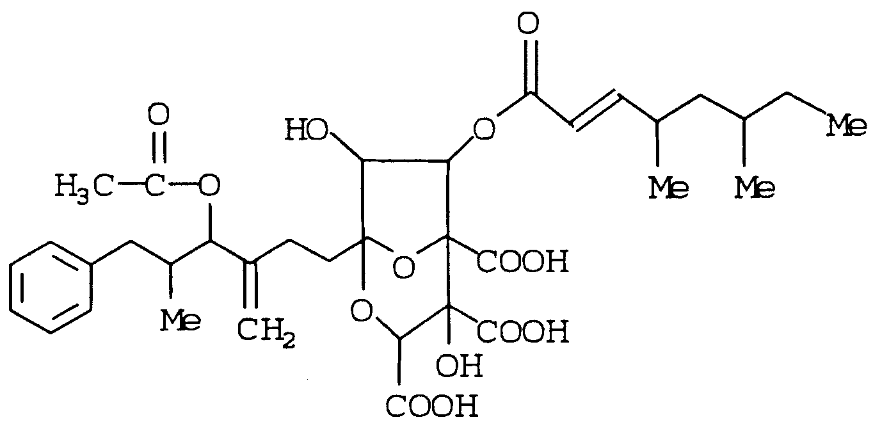

(I)

in recoverable amounts.

3. A biologically pure culture of Claim 1 which is MF5565 (ATCC 74068), or an active mutant thereof.

4. A culture of Claim 2 which is MF5565 (ATCC 74068), or an active mutant thereof.

5. A process for making a compound of structure:

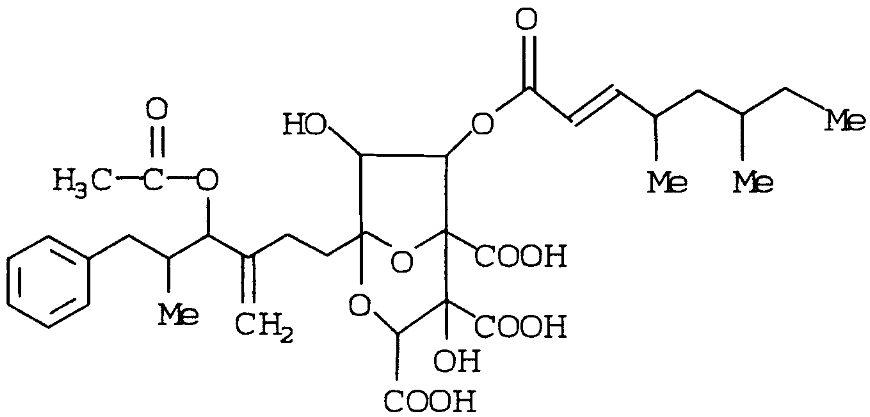

(I)

comprising cultivating Exserohilum rostratum under conditions suitable for formation of the compound and recovering the compound.

6. A process of Claim 5 wherein the cultivation is carried out for 14-35 days.

7. A process of Claim 5 wherein the strain of Exerohilum rostratum is MF5565 (ATCC 74068).

8. A process of Claim 7 wherein the cultivation is carried out for 14-35 days.

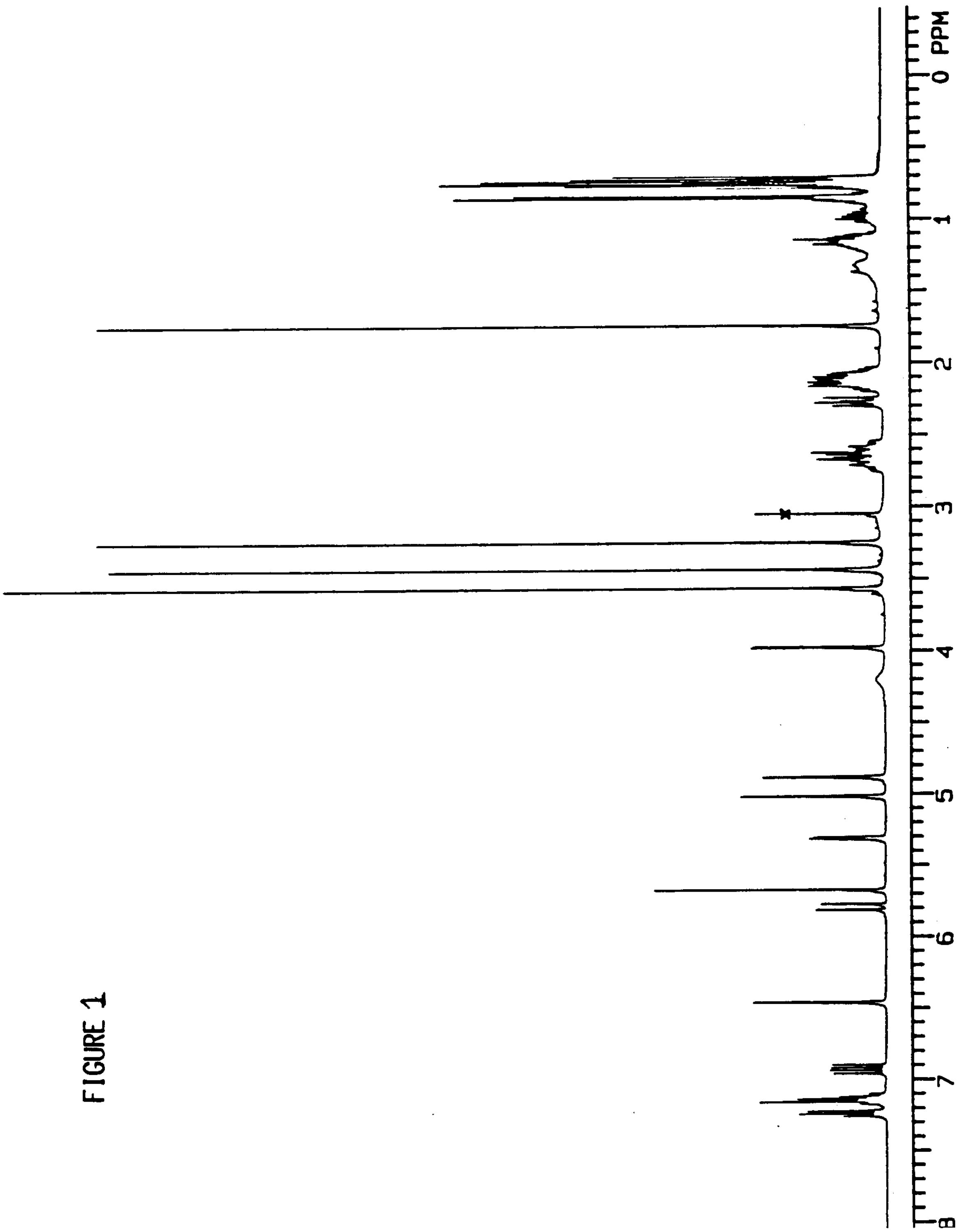


FIGURE 1

European Patent Office

# EUROPEAN SEARCH REPORT

Application Number

EP 92 20 1809

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-5 026 554 (K.F.BARTIZAL ET AL.)<br>* the whole document *<br>--- | 1-8 | C12N1/14<br>C12P7/40<br>//(C12P7/40,<br>C12R1/645) |
| A | BIOLOGICAL ABSTRACTS<br>, 1989, Philadelphia, PA, US;<br>abstract no. 98350,<br>LEONARD K J; THAKUR R P; LEATH S<br>'INCIDENCE OF BIPOLARIS AND EXSEROHILUM SPECIES IN CORN LEAVES IN NORTH CAROLINA USA.'<br>* abstract *<br>& PLANT DIS 72 (12). 1988. 1034-1038<br>--- | 1-8 | |
| P,X | EP-A-0 450 812 (MERCK & CO.INC.)<br>* the whole document *<br>----- | 1-8 | |
| | | | TECHNICAL FIELDS SEARCHED (Int. Cl.5) |
| | | | C12P |

The present search report has been drawn up for all claims

| Place of search | Date of completion of the search | Examiner |
|---|---|---|
| BERLIN | 23 OCTOBER 1992 | GURDJIAN D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)